# EUROPÄISCHE PATENTSCHRIFT

(11) **EP 3 171 691 B1**
(45) Veröffentlichungstag und Bekanntmachung des Hinweises auf die Patenterteilung: **26.08.2020**
(21) Anmeldenummer: 15745171.7
(22) Anmeldetag: 22.07.2015
(51) Int. Cl.: A01K 61/00, A01G 33/00, A01K 63/04, C02F 3/00

(54) **AQUAKULTURANLAGE UND KULTIVIERUNGSVERFAHREN**
AQUACULTURE INSTALLATION AND FARMING METHOD
INSTALLATION D'AQUACULTURE ET PROCÉDÉ DE PISCICULTURE

(30) Priorität: 23.07.2014 DE 202014103397 U
(43) Veröffentlichungstag der Anmeldung: 31.05.2017
(73) Patentinhaber: Bock, Gordon, 64395 Brensbach (DE); Linke, Rainer, 53175 Bonn (DE)
(72) Erfinder: BOCK, Gordon, 64395 Brensbach (DE)
(74) Vertreter: Ernicke, Klaus Stefan
(86) Internationale Anmeldenummer: PCT/EP2015/066732
(87) Internationale Veröffentlichungsnummer: WO 2016/012489

(56) Entgegenhaltungen:
- FR-A1- 2 471 138
- FR-A1- 2 627 349
- JP-A- 2002 010 723
- US-A1- 2009 145 368
- US-A1- 2012 312 244
- US-A1- 2013 149 411

## Beschreibung

Die Erfindung betrifft eine Aquakulturanlage und ein Kultivierungsverfahren.

Aus der US 2013/149411 A1 sind eine Aquakulturanlage und ein Kultivierungsverfahren bekannt, die eine Aquakulturanlage mit einem Fischzuchtbehälter und einem Behälter mit einem Zwischen-Tiersystem einschließlich Würmern sowie einen Biomassereaktor umfassen. Der Biomassereaktor hat eine Doppelfunktion betreffend eine biologische Degradation von pflanzlichen Abfällen mittels Mikroben und eine Filterung von partikulären Ausscheidungen der gezüchteten Fische mittels üblicher Abfallwasserbehandlungssysteme, einschließlich eines Klärsystems. Aus dem Biomassereaktor wird gereinigtes und klares Wasser an das nachgeschaltete Zwischen-Tiersystem abgegeben.

Eine DE 10 2012 012 259 B3 zeigt eine andere Aquakulturanlage. Sie weist ein Fischbecken auf, dem ein mechanischer Filter und ein biologischer Filter mit Mikroorganismen zur Aufbereitung des Kulturwassers nachgeschaltet sind.

Die FR 2 627 349 A befasst sich mit einem Transportbehälter für geerntete Muscheln oder Austern. Diese liegen offen auf einer abwärts geneigten Platte und werden durch das zeitweise zugeführte Wasser einerseits frisch gehalten und können sich andererseits selbst reinigen.

Es ist Aufgabe der vorliegenden Erfindung, eine verbesserte Aquakulturtechnik aufzuzeigen.

Die Erfindung löst diese Aufgabe mit den Merkmalen im Verfahrens- und Vorrichtungshauptanspruch.

Die beanspruchte Aquakulturtechnik, d.h. die Aquakulturanlage und das zugehörige Kultivierungsverfahren, haben mehrere Vorteile hinsichtlich Funktion, Effizienz, Umweltverträglichkeit, Nachhaltigkeit und Wirtschaftlichkeit. Der Aufwand für die Fütterung der aufgezogenen aquatischen Lebewesen, insbesondere Fische, kann wesentlich reduziert werden. Der Wasserverbrauch ist ebenfalls sehr gering. Ein Einsatz von Antibiotika kann entfallen oder zumindest wesentlich reduziert werden.

Der mit dem Kulturbehälter für aquatische Lebewesen in Fluidverbindung stehende Behälter zur Aufzucht von Futtertieren kann mehrere Funktionen bzw. einen Mehrfachnutzen haben.

Die Futtertiere nehmen einerseits die partikulären Ausscheidungen der aquatischen Lebewesen oder auch sonstige ungelöste Partikel im Kulturwasser auf und erfüllen dadurch eine Filterfunktion als Futtertierfilter. Sie können außerdem ggf. Plankton und Mikroorganismen aus dem Kulturwasser filtern. Ferner ist eine Nahrungsaufnahme der Futtertiere von der Substratschicht mit Hilfe ihrer Kiefer möglich. Als Futtertiere können z.B. marine Würmer, insbesondere Borstenwürmer (Annelida) verwendet werden. Bevorzugt ist der Einsatz von Wattwürmern, insbesondere Seeringelwürmern (Nereididae).

Andererseits können die nahrungstechnisch unmodifizierten, insbesondere naturbelassenen und ggf. noch lebenden Futtertiere zum Füttern der aquatischen Lebewesen im Kulturbehälter genutzt werden. Auf eine nahrungstechnische Modifikation der Futtertiere durch Zerkleinerung, Aufbereitung oder Weiterverarbeitung, insbesondere Extrudieren, zu Fischfutter bzw. Trockenfutter kann verzichtet werden. Die Ernährung und Ernährungsweise der aquatischen Lebewesen kann dadurch wesentlich verbessert werden. Die naturbelassene und ggf. noch lebende Biomasse ist gesünder als nahrungstechnisch modifiziertes Fischfutter, insbesondere Fischmehl. Der bei marinen Würmern, insbesondere Borstenwürmern bzw. Seeringelwürmern, vorhandene Schleimmantel wirkt sich z.B. besonders gesundheitsfördernd aus. Borstenwürmer z.B. bilden in ihrem natürlichen Lebensraum einen wichtigen Bestandteil in der Ernährung vieler kommerzieller nutzbarer Fisch- und Krebstierarten. Als solche tragen sie zu einer besseren Verdaulichkeit und Stärkung des Immunsystems als modifizierte Futtermittel bei. Letztere bestehen in der Regel zu 90% aus Trockenmasse und weichen daher deutlich in ihrer Konsistenz von den in der Natur vorhandene Fischnährtieren ab.

Die Futterproduktion kann in den Wasserkreislauf der Aquakulturanlage integriert werden. Dies kann für die Verträglichkeit von Vorteil sein. Der gegenseitige Austausch von Stoffwechselprodukten (chemische Botenstoffe, Aminosäuren etc.) durch das gemeinsame Kulturwasser wirkt sich positiv auf das Wachstum und die Haltungsbedingungen der aquatischen Lebewesen aus.

Außerdem können durch die Einbindung in die Aquakulturanlage und die räumliche Nähe zum Kulturbehälter, insbesondere Fischtank, kurze Wege und Frische des Lebendfutters gewährleistet werden. Die Aquakulturanlage kann einen Filterkreislauf und/oder einen Fütterkreislauf bzw. eine Fütterkette haben. Die Kreisläufe können in Verbindung stehen. Die an die Futtertiere verfütterten Mikroalgen können auch in den Kulturbehälter gelangen und zur dortigen (passiven) Ernährung der aquatischen Lebewesen dienen.

Durch die integrierte Futterproduktion kann eine Fütterung der aquatischen Lebewesen mit nahrungstechnisch modifiziertem Futtermitteln entfallen oder signifikant verringert werden. Die beanspruchte Aquakulturtechnik zeichnet sich durch einen geringen Phosphatgehalt aus. Bei herkömmlichen Fischzuchten wird über das Fischfutter Phosphat in übermäßiger Weise dem Kulturwasser zugeführt. Außerdem kann bei der beanspruchten Aquakulturtechnik die bisher übliche aufwändige Regulierung der Wasserstabilität und des Nährstoffgehaltes durch Zugabe von künstlichen Stabilisatoren oder Bindemitteln und der Betrieb von mechanischen Filtern (Trommelfilter, Abschäumer, etc.) entfallen oder wesentlich verringert werden. Andererseits können in einer Substratschicht, insbesondere einer Sandschicht, des Futtertierfilters weitere Reinigungsprozesse des Kulturwassers stattfinden, wie z.B. Stickstoff-Entfernung, Mineralisation, Nitrifikation, Denitrifikation und Annamox.

Dem Futtertierfilter kann ein Filter für die im Kulturwasser gelösten Ausscheidungen der aquatischen Lebewesen zugeordnet, insbesondere nachgeschaltet sein. Hierfür eignet sich besonders ein Pflanzenfilter, insbesondere in der Ausführung als Hydrokultur bzw. Hydroponik. Wenn die Aquakulturanlage mit Salzwasser betrieben wird, kann durch eine Flutungseinrichtung eine meeresähnliche Umgebung mit Ebbe und Flut simuliert werden. Hierdurch können z.B. die Umgebungsbedingungen solcher salzwassertoleranter Pflanzen bzw. Halophyten optimiert werden. Die Pflanzen können verzehrt werden und bieten dadurch ebenfalls einen Mehrfachnutzen. Besonders geeignet ist Queller.

Eine solche Flutungseinrichtung und Simulation einer meeresähnlichen Umgebung mit Ebbe und Flut wird erfindungsgemäß bei den Behältern für die Aufzucht von Futtertieren bzw. beim Futtertierfilter eingesetzt.

Für den Fütterkreislauf der Futtertiere und der aquatischen Lebewesen ist außerdem die Einbindung eines Algenreaktors von Vorteil, der auch in den Kulturwasserkreislauf eingeschaltet werden kann. Mit dem Algenreaktor können Algen, insbesondere Mikroalgen, schnell und effizient produziert werden. Mit den Algen können die Futtertiere zusätzlich gefüttert werden. Dies beschleunigt deren Wachstumsprozess und erhöht auch die Qualität der Futtertiere. Die aquatischen Lebewesen bekommen aus den Futtertieren mehr und bessere Nährstoffe als aus einer üblichen Fütterung mit Trockenfutter. Diese Nährstoffe sind für die aquatischen Lebewesen auch besser verfügbar. Durch eine Fütterung mit nahrungstechnisch unmodifizierten Futtertieren und ggf. Mikroalgen können die aquatischen Lebewesen, insbesondere Fische, bevorzugt Seezungen, schneller wachsen und in der Nahrung enthaltene Nährstoffe besser verwerten. Die Gesundheit und die Verzehrqualität der aquatischen Lebewesen werden deutlich verbessert.

Die verschiedenen Behälter für die aquatischen Lebewesen, die Futtertiere, die Pflanzen und der Algenreaktor können untereinander durch einen Wasserkreislauf mit dem Kultur- oder Prozesswasser verbunden sein. An geeigneten Stellen kann verbrauchtes Wasser abgezogen und Frischwasser, z.B. echtes oder künstliches Meerwasser, eingespeist werden, was vorzugsweise im Zulauf für den Algenreaktor erfolgt. Damit kann der Verlust von lebenswichtigen Nährstoffen und Spurenelementen im Kulturwasser nach der Entnahme bzw. Ernte von aquatischen Lebewesen und ggf. Pflanzen ausgeglichen werden. Ferner kann eine Wasseraufbereitung, insbesondere eine Sauerstoffanreicherung, des Kulturwassers erfolgen, was bevorzugt im Zulauf oder direkt im Kulturbehälter geschieht.

In den Wasserkreislauf können eine oder mehrere Pumpensümpfe mit zugehörigen Pumpen eingebunden sein. Vorzugsweise sind zwei oder mehr Pumpensümpfe vorhanden. Die Pumpensümpfe können der bedarfsgerechten Verteilung des Kulturwassers an ggf. verschiedene Abnehmer dienen. Sie sind vorzugsweise an mehrere Fluidverbindungen, insbesondere rohrförmige Leitungen, angeschlossen. Hierbei kann eine Volumenstrom Steuerung stattfinden, die den ggf. unterschiedlichen Kapazitäten und Retentionszeiten der verschiedenen Filter, des Algenreaktors und anderen Gegebenheiten Rechnung trägt.

Die genannten Behälter können jeweils mehrfach vorhanden sein. Für die Behälter zur Aufzucht von Futtertieren und für die Pflanzen empfiehlt sich jeweils eine Turm- oder Kaskadenanordnung, wobei das Kulturwasser die verschiedenen Behälter nacheinander und durch Schwerkraft durchfließt. Der Energie- und Pumpaufwand kann reduziert werden. Außerdem lassen die Behälter sich platz- und kostensparend in einem Regal unterbringen. Dies erleichtert auch die Handhabung bei der Zuchtpflege und der Ernte sowie der Instandhaltung.

Im Behälter zur Aufzucht von Futtertieren und ggf. Kulturbehälter für die aquatischen Lebewesen sind Substratschichten, insbesondere Sandschichten und/oder Kiesschichten, bevorzugt bodenseitig vorhanden. Im Kulturbehälter können diese Substratböden einen Teil der Futtertiere beherbergen, die dadurch in gewohnter Umgebung weiterleben können und für die aquatischen Lebewesen, insbesondere gründelnde Fische oder Garnelen, eine längerfristige und gut zugängliche Futterquelle bilden. Diese Behälter können einen optimierten Fluidablauf besitzen. Dieser kann getrennte und/oder optimierte Auslassöffnungen für das Kulturwasser und/oder das Substrat besitzen. Er ermöglicht einerseits einen permanenten und dabei kontrollierten Ablauf von Kulturwasser. Andererseits erlaubt er auf einfache Weise einen Abzug der Substratschicht und der darin enthaltenen Futtertiere.

Die beanspruchte Aquakulturanlage hat ferner den Vorteil eines besonders einfachen konstruktiven und kostengünstigen Aufbaus und Betriebs. Hierbei ist die Anordnung der Behälter in Stapeln von Vorteil. Gleiches gilt für den bevorzugt vertikalen Aufbau der Aquakulturanlage und die Unterbringung in einem Regal.

Der Algenreaktor und dessen Ausbildung hat eigenständige erfinderische Bedeutung und kann auch bei anderen Aquakulturanlagen eingesetzt werden. Hier sind die Funktionen der Algenzucht unter Lichtzufuhr und Photosynthese sowie der Energieverwertung und der Regeneration optimiert.

In den Unteransprüchen sind weitere vorteilhafte Ausgestaltungen der Erfindung angegeben.

Die Erfindung ist in den Zeichnungen beispielhaft und schematisch dargestellt. Im Einzelnen zeigen:
- Figur 1:: eine perspektivische Ansicht einer Aquakulturanlage mit mehreren Behältern in einem Regal,
- Figur 2:: einen schematischen hydraulischen Schaltplan der Aquakulturanlage,
- Figur 3:: einen Zuordnungs- und Schaltplan der Aquakulturanlage,
- Figur 4:: einen Wurmfilter,
- Figur 5:: einen Kulturbehälter für Fische,
- Figur 6:: einen Pflanzenfilter,
- Figur 7:: einen Algenreaktor und
- Figur 8:: ein Reaktormodul für den Algenreaktor von Figur 7.

Die Erfindung betrifft eine Aquakulturanlage (1) und ein Kultivierungsverfahren für aquatische Lebewesen (2). Die Erfindung betrifft ferner einen Algenreaktor (9).

Die aquatischen Lebewesen sind im gezeigten und bevorzugten Ausführungsbeispiel Fische, insbesondere Raubfische. Dies können Salzwasserfische oder Süßwasserfische sein. Alternativ oder zusätzlich können die aquatischen Lebewesen (2) Weichtiere (Mollusca), z.B. Austern, Miesmuscheln etc. oder Krebstiere (Crustacea), z.B. Garnelen, sein. Die Aquakulturanlage (1) dient zur Aufzucht von solchen aquatischen Lebewesen (2). Die zyklische Zuchtdauer für Salzwasserfische (2), z.B. Seezungen, beträgt z.B. zwischen 1-2 Jahren.

Die Aquakulturanlage (1) weist einen Kulturbehälter (6), insbesondere Fischtank, mit Kulturwasser (5) oder Prozesswasser zur Aufnahme der aquatischen Lebewesen (2) auf. Das Kulturwasser (5) kann Salzwasser oder Süßwasser sein. Im gezeigten Ausführungsbeispiel wird Salzwasser bevorzugt. Der Kulturbehälter (6) hat z.B. eine kubische Form. Aus hydraulischen Gründen kann auch eine konische oder zylindrische Form oder ein Kubus mit gerundeten Eckbereichen gewählt werden. Der Kulturbehälter (6) kann aus einem geeigneten Material, z.B. Kunststoff oder Metall, Holz oder Beton, bestehen.

Der Kulturbehälter (6) kann an der Oberseite offen sein. Am Boden des Kulturbehälters (6) kann sich eine Substratschicht (28), z.B. Sand, befinden. Der Zulauf und Ablauf (33,29) für das Kulturwasser (5) sind an Fluidverbindungen (12), z.B. geschlossene Leitungen in Form von Rohren oder Schläuchen, angeschlossen. Alternativ sind bereichsweise offene Leitungen oder Kanäle möglich.

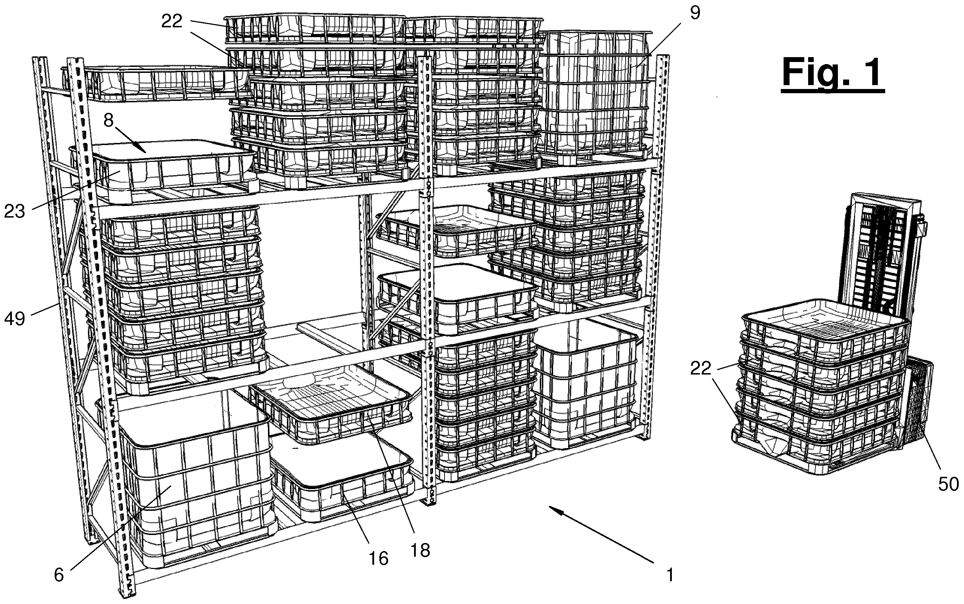

Wie Figur 1 bis 3 verdeutlichen, weist die Aquakulturanlage (1) außerdem einen Behälter (22) für Futtertiere (3) auf und kann einen Behälter (23) für Pflanzen (4) sowie einen Algenreaktor (9) aufweisen. Die Behälter (6,22,23) und ggf. der Algenreaktor (9) können jeweils mehrfach vorhanden sein. Sie können dabei in einem Turm oder Rack mit Distanz oder in einem Stapel mit gegenseitiger Berührung und Abstützung angeordnet sein. Die Behälter (6,22,23) oder Behälterstapel und ggf. der Algenreaktor (9) können z.B. auf einer Palette stehen. Die Formen und Abmessungen der Behälter (6,22,23) können ähnlich sein, insbesondere hinsichtlich der Grundfläche. Die Behälter (22,23) sind dabei flacher bzw. niedriger als der Kulturbehälter (6). Die Grundmaße sind bevorzugt standardisiert. Die Grundfläche ist z.B. an das Maß einer Europalette angepasst.

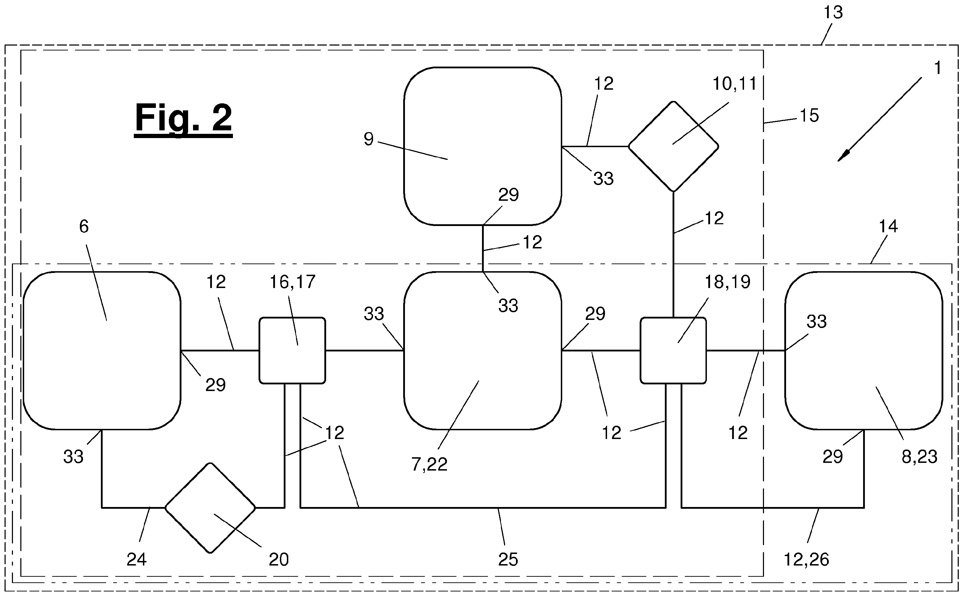

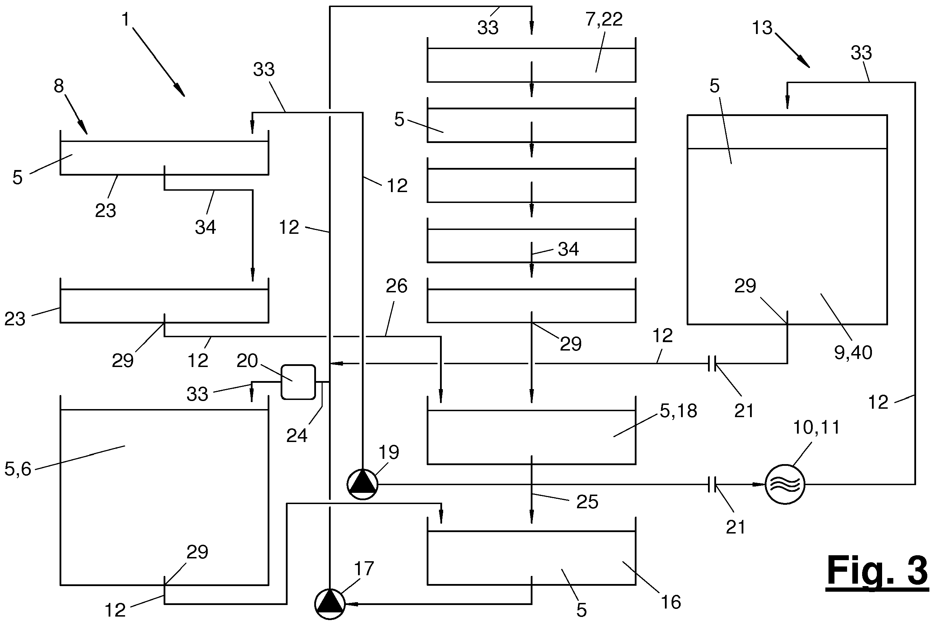

Die Behälter (6,22,23) und der Algenreaktor (9) können miteinander hydraulisch in einem Wasserkreislauf für das Kulturwasser oder Prozesswasser (5) verbunden sein. Figur 2 und 3 zeigen hierzu beispielhaft hydraulische Schaltpläne. Figur 1 zeigt schematisch die Aquakulturanlage (1) mit der Behälteranordnung ohne Fluidverbindungen und Pumpen.

Der Kulturbehälter (6) mit den aquatischen Lebewesen (2) ist über eine Fluidverbindung (12) des Kulturwassers (5) mit einem Behälter (22) zur Aufzucht von Futtertieren (3) verbunden. Wenn mehrere dieser Behälter (22) vorhanden sind, können diese untereinander durch eine Fluidüberleitung (34) hydraulisch verbunden sein. Die Futtertiere (3) dienen zur Fütterung und Versorgung der aquatischen Lebewesen (2) mit Lebendfutter. Die Futtertiere (3) sind z.B. marine Würmer, vorzugsweise Borstenwürmer bzw. Seeringelwürmer.

Der Behälter (22) mit den Futtertieren (3) bildet einerseits einen Futtertierfilter (7), insbesondere einen Wurmfilter, welcher partikuläre Stoffe aus dem Kulturwasser (5) filtert. Die partikulären Stoffe können partikuläre Ausscheidungen der aquatischen Lebewesen (2) im Kulturbehälter (6), Futterreste oder sonstige Feststoffe sein. Sie werden von den Futtertieren (3) aufgenommen und aus dem Kulturwasser (5) entfernt. Insbesondere Würmer (3), vor allem Borstenwürmer bzw. Seeringelwürmer, können mit diesen partikulären Stoffen gefüttert werden. Der Futtertierfilter (7) ist mit dem Kulturbehälter (6) in einem Filterkreislauf (14) des Kulturwassers (5) verbunden.

Der Behälter (22) bzw. der Futterfilter (7) kann über eine Fluidverbindung (12) mit einem Filter (8) für gelöste Stoffe im Kulturwasser (5), insbesondere gelöste Ausscheidungen der aquatischen Lebewesen (2), verbunden sein. Der Filter (8) ist in den Filterkreislauf (14) eingebunden. Er ist dabei vorzugsweise in Strömungsrichtung dem Futterfilter (7) nachgeschaltet.

Der Filter (8) ist in den gezeigten und bevorzugten Ausführungsbeispielen als Pflanzenfilter ausgebildet. Alternativ kann er in anderer Weise, z.B. als biologischer Filter mit Mikroorganismen, ausgestaltet sein.

Der Behälter (22) mit den Futtertieren (3) und der Kulturbehälter (6) sind ferner in einem Fütterkreislauf (15) verbunden. Die Futtertiere (3) dienen als Lebendfutter zur Ernährung der aquatischen Lebewesen (2) und werden in geeigneter Weise in den Kulturbehälter (6) überführt.

Der Algenreaktor (9) kann ebenfalls in den Fütterkreislauf (15) eingebunden sein. Er ist bevorzugt als Photobioreaktor für die Mikroalgenzucht ausgebildet. Im Algenreaktor (9) werden Algen, insbesondere Mikroalgen, in einer Algensuspension (40) gezüchtet. Den Algen können Nährstoffe und Licht für die Photosynthese zugeführt werden. Der Algenproduktionszyklus kann z.B. 1 bis 2 Wochen dauern.

Die Algensuspension (40) kann den Futtertieren (3) für deren Ernährung über den Wasserkreislauf (13) zugeführt werden. Die Futtertiere (3) und die damit gefütterten aquatischen Lebewesen (2) können mittels der Algen auf natürlichem und artgerechtem Weg gesund und ohne Schadstoffe sowie kostengünstig ernährt werden. Bedarfsweise kann den aquatischen Lebewesen (2) und den Futtertieren (3) anderes und zusätzliches Futter zugeführt werden.

Figur 2 verdeutlicht einen hydraulischen Schaltplan für die Verbindung von Kulturbehälter (6), Futterfilter (7), Filter (8) und Algenreaktor (9). Das Kulturwasser (5) wird in der Aquakulturanlage (1) in einem geschlossenen Wasserkreislauf (13) geführt. Bedarfsweise kann dabei an einer Wasserzufuhr (10) Frischwasser zugeführt werden. An einer Wasserabfuhr (11) kann verbrauchtes Kulturwasser (5) aus dem Kreislauf (13) entnommen werden. Ferner kann im Wasserkreislauf (13) eine Wasseraufbereitung (20) angeordnet sein. Dies kann z.B. eine Sauerstoffanreicherung sein.

Für die Umwälzung des Kulturwassers (5) im Wasserkreislauf (13) sind ein oder mehrere Pumpensümpfe (16, 18) mit ein oder mehreren zugeordneten Pumpen (17, 19) vorgesehen. An einen Pumpensumpf (16, 18) können mehrere Fluidverbindungen (12) und mehrere Abnehmer bzw. Komponenten (6,7,8,9) der Aquakulturanlage (1) angeschlossen sein. Die Fluidverbindungen (12) zwischen den verschiedenen Komponenten der Aquakulturanlage (1) werden durch die besagten, vorzugsweise geschlossenen Leitungen, gebildet.

Wie Figur 2 verdeutlicht ist der Kulturbehälter (6) ablaufseitig über eine Fluidverbindung (12) mit einem ersten Pumpensumpf (16) eingangseitig verbunden. Ausgangseitig weist der Pumpensumpf (16) mehrere Fluidverbindungen (12) auf. Er ist einerseits über eine Rückführleitung (24) mit dem Fluidzulauf (33) des Kulturbehälters (6) verbunden. In der Rückführleitung (24) kann die erwähnte Wasseraufbereitung (20) angeordnet sein. Ferner ist der Pumpensumpf (16) über eine Fluidverbindung (12) mit dem Futtertierfilter (7) zulaufseitig verbunden.

Ablaufseitig ist der Futtertierfilter (7) mit einem zweiten Pumpensumpf (18) verbunden. Aus diesem Pumpensumpf (18) wird der Filter (8), insbesondere Pflanzenfilter, gespeist. Das Kulturwasser (5) kann aus dem Filter (8) über eine Rückführleitung (26) in den zweiten Pumpensumpf (18) zurückfließen. Dies kann z.B. durch Schwerkraft geschehen. Ferner kann das Kulturwasser (5) aus dem zweiten Pumpensumpf (18) über eine Rückführleitung (25) am Futtertierfilter (7) vorbei zum ersten Pumpensumpf (16) gefördert werden.

Der Algenreaktor (9) ist ebenfalls über eine Fluidverbindung (12) an den zweiten Pumpensumpf (18) zulaufseitig angeschlossen. In dieser Fluidverbindung (12) kann sich der vorerwähnte Zu- und Ablauf (10, 11) befinden. Ablaufseitig ist der Algenreaktor (9) mit dem Futtertierfilter (7) verbunden.

Ein Teil der Fluidverbindungen (12) kann von aktiven Förderleitungen gebildet werden, in den Pumpendruck ansteht. Andere Fluidverbindungen (12), insbesondere die Rückleitungen (24,25,26) können passive Förderleitungen sein, in denen das Kulturwasser (5) durch Schwerkraft fließt.

Figur 3 zeigt das hydraulische Schaltschema von Figur 2 und zusätzlich die räumliche Zuordnung der Komponenten der Aquakulturanlage (1). Hierbei sind auch eine oder mehrere diskontinuierliche Fluidverbindungen (21) ersichtlich, durch die der Wasserstrom zeitweise abgesperrt werden kann. Eine solche diskontinuierliche Fluidverbindung (21) ist z.B. an den zulauf- und ablaufseitigen Fluidverbindungen (12) des Algenreaktors (9) angeordnet.

Wie Figur 3 verdeutlicht, kann der Kulturwassertransport durch den Futtertierfilter (7) und/oder durch den Filter (8), insbesondere Pflanzenfilter, durch Schwerkraft und in einer kaskadenförmigen Filteranordnung erfolgen. Die Pumpe (17) fördert das Kulturwasser (5) aus dem ersten Pumpensumpf (16) über eine Fluidverbindung zum Futtertierfilter (7), insbesondere zu dessen oberen Fluidzulauf (33). Von dieser Fluidverbindung (12) zweigt die Rückführleitung (24) zum Kulturbehälter (6) und dessen Fluidzulauf (33) ab. Vom Kulturbehälter (6) kann das Kulturwasser über den Fluidablauf (29) durch Schwerkraft zum tiefer liegenden ersten Pumpensumpf (16) fließen.

Der zweite Pumpensumpf (18) ist oberhalb des ersten Pumpensumpfes (16) und unterhalb des Futtertierfilters (7) angeordnet, wobei seine Pumpe (19) das Kulturwasser (5) zum Filter (8), insbesondere Pflanzenfilter und bevorzugt zu dessen oberen Fluidzulauf (33) fördert. Über die Rückführleitung (26) fließt das Kulturwasser (5) ablaufseitig aus dem Filter (8) durch Schwerkraft wieder zum zweiten Pumpensumpf (18).

Die Pumpe (19) kann andererseits über eine entsprechende Ventilanordnung und diskontinuierliche Fluidverbindung (21) Kulturwasser (5) zulaufseitig in den Algenreaktor (9) pumpen. Von hier kann die Algensuspension (40) durch Schwerkraft direkt zum Futtertierfilter (7) oder in dessen Fluidverbindung (12) vom ersten Pumpensumpf (16) fließen.

Die Wasserströme innerhalb der Aquakulturanlage (1) können unterschiedlich groß sein, wobei die Verteilung über den oder die Pumpensümpfe (16, 18) in Verbindung mit entsprechenden Steuermitteln, insbesondere Ventilen, erfolgt. Zwischen dem Kulturbehälter (6) und dem ersten Pumpensumpf (16) werden z.B. 1.000 l/h über die Rückleitung (24) umgepumpt. Dem Behälter (22) bzw. Futtertierfilter (7) wird wegen der Retentionszeit ein demgegenüber verringerter Volumenstrom von z.B. 300 l/h zugeführt und nach Durchlaufen in den zweiten Pumpensumpf (18) geleitet. Der hiervon dem Pflanzenfilter (8) zugeführte Volumenstrom kann noch stärker verringert sein. Er kann z.B. 100 1/h betragen. Die restlichen 200 l/h können über die Rückleitung (25) wieder dem ersten Pumpensumpf (16) zugeführt werden.

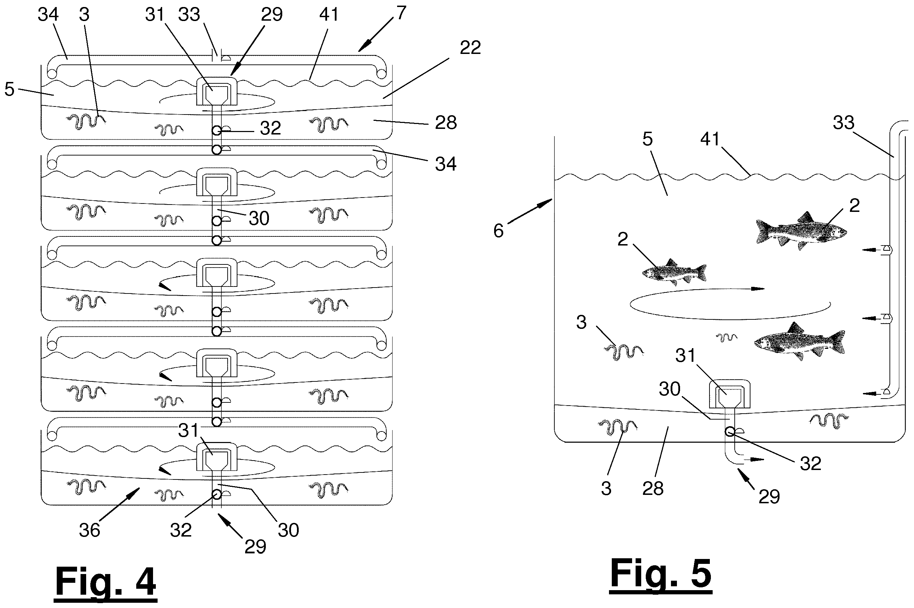

Figur 4 verdeutlicht den Aufbau des Futtertierfilters (7). Die ein oder mehreren Behälter (22) sind jeweils als offene und wannenartige Behälter ausgeführt, an deren Boden sich eine Substratschicht (28) mit Futtertieren (3), insbesondere Borstenwürmern bzw. Seeringelwürmern, befindet. Das Kulturwasser (5) überdeckt die Substratschicht (28).

Der oder die Behälter (22) weisen jeweils einen Fluidzulauf (33) und einen Fluidablauf (29) auf. Bei der gezeigten Turm- oder Kaskadenanordnung können der Fluidablauf (29) und der Fluidzulauf (33) benachbarter Behälter (22) gekoppelt sein und einen Fluidüberlauf (34) mit Schwerkraftförderung des Kulturwassers (5) bilden. Der Fluidzulauf (33) besteht z.B. jeweils aus einer zentralen Zuführleitung und einer Verzweigung mit mehreren Zuführsträngen, die das Kulturwasser verteilt in den jeweiligen Behälter (22) einspeisen.

Der Fluidablauf (29) weist ein in den Behälter (22) ragendes Ablaufrohr (30) auf, das an einem Ende mit einem Siphon (31) verbunden ist, der oberhalb der Substratschicht (28) bevorzugt zentral im Wasserbad angeordnet ist. Am anderen Ende ist das Ablaufrohr (30) mit einer tiefliegenden und im Bodenbereich des Behälters (22) angeordneten Auslauföffnung (32) verbunden. Die Verbindung kann lösbar sein, so dass bei Abzug des Ablaufrohrs (30) die Substratschicht (28) aus der Auslauföffnung (32) abfließen kann.

In der Auslauföffnung (32) kann ein Gitter oder ein anderes Rückhalteelement vorhanden sein, welches einen Austritt der Futtertiere (3) verhindert. Nach Ablauf der Substratschicht (28) können die Futtertiere (3) geerntet und als Lebendfutter in den Kulturbehälter (6) zugeführt werden. Aus dem gezeigten Stapel von Futtertierbehältern (22) kann z.B. einmal täglich geerntet werden.

In einer anderen Variante können für das Kulturwasser (5) und für die Substratschicht (28) eigene und räumlich getrennte Auslassöffnungen (32) vorhanden sein. Diese können auch eigenständig geöffnet und geschlossen werden.

Figur 5 zeigt den mit Kulturwasser (5) und mit aquatischen Lebewesen (2) gefüllte Kulturbehälter (6). Die lebenden Futtertiere (3) werden in das Kulturwasser (5) gegeben und können dort schweben oder schwimmen und von den aquatischen Lebewesen (2) gefressen werden. Am Behälterboden ist ebenfalls eine Substratschicht (28), insbesondere eine Sandschicht, angeordnet. Diese kann ebenfalls Futtertiere (3), insbesondere Borstenwürmer bzw. Seeringelwürmer, enthalten. Gründelnde Fische (2) können die Substratschicht (28) durchwühlen und die dortigen Futtertiere (3) fressen.

Der Kulturbehälter (6) weist einen Fluidzulauf (33) auf. Dieser kann ein Tauchrohr mit mehreren Auslassöffnungen besitzen. Am Behälterboden ist ein Fluidablauf (29) angeordnet. Dieser kann in gleicher Weise wie bei den Behältern (22) und beim Futtertierfilter (7) ausgebildet sein.

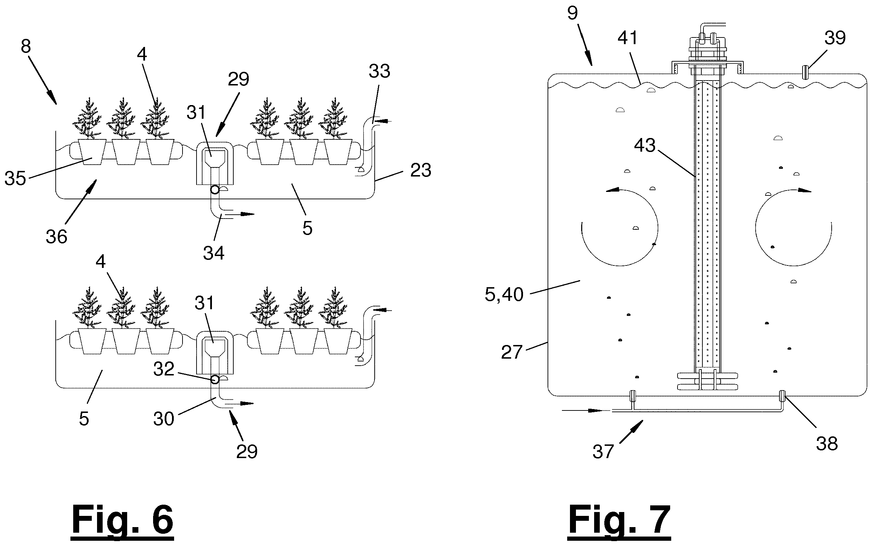

In Figur 6 ist ein Pflanzenfilter (8) dargestellt. Er weist mehrere an der Oberseite offene, wannenartige Behälter (23) auf, die mit einem Fluidzulauf (33) und einem Fluidablauf (29) versehen sind. Die Pflanzen (4) sind an das jeweilige Kulturwasser (5) angepasst.

In dem gezeigten Ausführungsbeispiel kommen salztolerante Pflanzen bzw. Halophyten, insbesondere Queller, zum Einsatz, die in einer Hydroponik (35) oder Hydrokultur im Behälter (23) gehalten sind. Die Pflanzen (4) wurzeln in einem anorganischen Substrat, welches in wasserdurchlässigen Pflanztöpfen gehalten ist. Die Pflanzenwurzeln können dadurch vom Kulturwasser (5) umströmt werden. Sie können die im Kulturwasser (5) enthaltenen gelösten Ausscheidungen als Nährstoffe aufnehmen und das Wasser reinigen.

Die Pflanzen (4) können Nutz- und Speisepflanzen sein, z.B. Queller. Sie werden von den im Kulturwasser (5) gelösten Ausscheidungen ernährt und können zu gegebener Zeit geerntet werden. Bei Süßwasserbetrieb werden andere geeignete Pflanzen (4), z.B. Gemüse- oder Salatpflanzen, verwendet.

Der Fluidablauf (29) der Pflanzenbehälter (23) kann in ähnlicher Weise wie beim Kulturbehälter (6) und beim Futtertierbehälter (22) ausgebildet sein.

Der Pflanzenfilter (8) bzw. der oder die Behälter (23) können eine Flutungseinrichtung (36) aufweisen, mit der der Wasserstand relativ zu den Pflanzen (4) verändert werden kann. Hierdurch kann für salztolerante Pflanzen abwechselnd Ebbe und Flut simuliert werden, wobei die Pflanzen (4) nicht oder nur nur im Wurzelbereich und zeitweise vollständig von Kulturwasser (5) umspült werden.

Die Behälter (22) zur Aufzucht von Futtertieren (3) bzw. der Futtertierfilter (7) weisen ebenfalls eine solche Flutungseinrichtung (36) auf. Hierdurch kann eine meeresähnliche Umgebung mit Ebbe und Flut für die Futtertiere (3), insbesondere die marinen Würmer, simuliert werden.

Die Flutungseinrichtung (36) kann auf unterschiedliche Weise den relativen Wasserstand ändern. In der gezeigten Ausführungsform sind z.B. die Pflanztöpfe der Hydroponik (35) untereinander durch einen Träger verbunden und können in ihrer Höhenlage innerhalb des Behälters (23) mit einem steuerbaren Stellantrieb verändert werden. In einer anderen Ausführungsform kann der Wasserstand (41) erhöht und gesenkt werden. In einer dritten Variante ist eine beiderseitige Verstellung möglich.

Eine Erhöhung und Senkung des Wasserstands (41) in den verschiedenen gestapelten Behältern (22,23) bzw. in der Turm- oder Kaskadenanordnung bei den Anordnungen von Figur 4 und 6 ist z.B. in folgender Weise möglich. Durch den Fluidzulauf (33) wird kontinuierlich Kulturwasser (5) in den Behälter (22,23) gepumpt, bis der Wasserspiegel (41) den Überlaufpunkt des Siphons (31) erreicht. Wird dieser Punkt überschritten, bildet sich im Ablaufrohr (30) durch den Unterdruck ein Saughebereffekt aus. Dieser bewirkt, dass das Kulturwasser (6) aus dem Behälter (22,23) schneller entleert wird, als es neu befüllt werden kann. Sobald der Wasserspiegel (41) bis zu einem kritischen Punkt abgesunken ist, zieht der Siphon (31) Luft und der Saughebereffekt bricht zusammen. Danach beginnt das Prinzip von neuem.

Die bodenseitigen Substratschichten (28) bzw. Hydrokulturschichten weisen ein Gefälle von z.B. 8% auf. An ihrem niedrigsten Punkt befindet der Siphon (31) am Ende des in den Behälter (22,23) ragenden Ablaufrohrs (30). Dies ermöglicht die weitgehend restlose Entleerung des Kulturwassers (5) aus dem Behälter (22,23) und die kurzzeitige Trockenlegung des Bodensubstrates. Dieser Vorgang ist für die Sauerstoffanreicherung des Substrates und die optimale Funktion des Wurmfilters (7) oder Pflanzfilters (8) bedeutsam.

Figur 7 verdeutlicht den Aufbau eines Algenreaktors (9). Dieser weist ein Reaktorgehäuse (27) auf, das an der Oberseite eine Zugangsöffnung haben kann, die mit einem Behälterdeckel (42) verschlossen wird. Der Reaktorbehälter (27) ist mit einer Mikroalgen-Suspension (40) und mit Wasser vorzugsweise mit Kulturwasser (5), gefüllt.

Der Reaktorbehälter (27) kann eine Belüftungseinrichtung (37) mit einer bevorzugt bodenseitigen Luftzufuhr (38) und einer bevorzugt deckelseitigen Luftabfuhr (39) aufweisen. Die Luftzufuhr (37) kann eine oder mehrere in den Behälterinnenraum mündete Luftdüsen aufweisen. Durch die Belüftung wird die Algensuspension (40) in der in Figur 7 angedeuteten Weise im Reaktorbehälter (27) bewegt und insbesondere umgewälzt. Die Luftabfuhr (39) kann als stellbares oder steuerbares Ablassventil ausgebildet sein. Die Algensuspension (40) füllt vorzugsweise nicht den gesamten Behälterinnenraum aus, wobei noch Luft zwischen der Behälteroberseite und dem Wasserspiegel (41) ist.

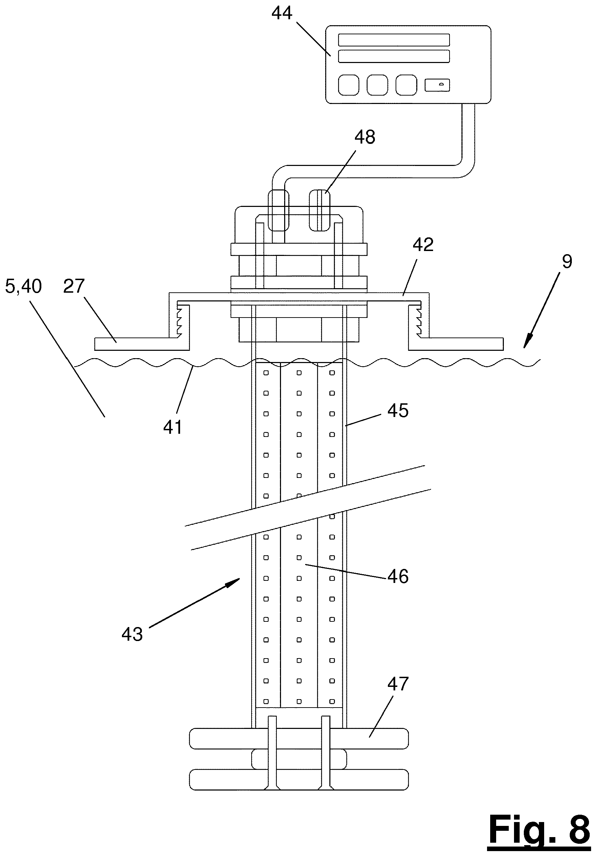

Der Algenreaktor (9) beinhaltet ein steuerbares Reaktormodul (43), das einzeln oder mehrfach vorhanden sein kann und das in die Algensuspension (40) eingetaucht werden kann. Das in Figur 7 und 8 dargestellte Reaktormodul (43) weist ein lichtdurchlässiges Rohr (45) mit einer darin angeordneten steuerbaren Leuchteinheit (46) auf. Das Rohr (45) ist z.B. als Glasrohr ausgebildet. Die Leuchteinheit (46) kann eine oder mehrere LED-Anordnungen, insbesondere LED-Platinen, aufweisen, die sich entlang des Rohrs (45) erstrecken und in die Algensuspension (40) strahlen. Das Reaktormodul (43) kann an geeigneter Stelle, z.B. am unteren freien Ende des Rohrs (45) einen Kühlkörper (47) aufweisen. Der Kühlkörper (47) kann die in der Leuchteinheit (46) entstehende Wärme in den Reaktorbehälter (27) und an die Algensuspension (40) ableiten. Er ist hierzu mit der Leuchteinheit (46) verbunden und wird außenseitig von der Algensuspension (40) umspült. Durch die Verwendung von LED's ist die Abwärme moderat.

Das Reaktormodul (43) ist am oberen Ende des Rohres (45) mit dem Behälterdeckel (42) verbunden und ragt aus diesem ein Stück heraus. An das Reaktormodul (43) kann eine Steuerung (44) außenseitig angeschlossen sein. Hierüber kann auch die Energiezufuhr zu der Leuchteinheit (46) erfolgen. Ferner kann außenseitig am Reaktormodul (43) ein Überdruckventil (48) angeordnet sein, welches mit dem Rohrinnenraum verbunden ist. Das Rohr (45) ist gegenüber dem Kühlkörper (47) und dem Deckel (42) abgedichtet.

Das Reaktormodul (43) kann an geeigneter Stelle auch eine Sensorik zur Detektion und Überwachung des Algenwachstums und/oder der Umgebungsbedingungen aufweisen. Dies kann z.B. ein Temperatursensor sein. Die Algensuspension (40) kann im Algenreaktor (9) über die Steuerung (44) klimatisiert werden. Hierfür kann zum einen die Abwärme der Leuchteinheit (46) und zum anderen eine eventuelle Zusatzheizung oder auch eine Kühlung benutzt werden.

Wie Figur 1 verdeutlicht, sind die Komponenten (6,7,8,16,17,18,19,22,23) der Aquakulturanlage (1) in einem vertikalen Aufbau und in einem Regal (49) angeordnet. Die Behälter (6,22,23), insbesondere Behälterstapel, können auf Paletten angeordnet sein. Sie sind gut zugänglich und lassen sich mittels eines Fördermittels (50), z.B. eines Gabelstaplers, ein- und auslagern. Die Fluidverbindungen (12) können mittels geeigneter Schnellkupplungen und Sperrelement schnell geöffnet und geschlossen werden.

Abwandlungen der gezeigten und beschriebenen Ausführungsformen sind in verschiedener Weise möglich. Insbesondere können die Merkmale der vorbeschriebenen Ausführungsbeispiele und ihrer Abwandlungen beliebig miteinander kombiniert, insbesondere auch vertauscht werden.

Die gezeigte und optimierte Konfiguration der Aquakulturanlage (1) mit allen Komponenten, ggf. in Mehrfachanordnung, kann in unterschiedlicher Weise variiert, insbesondere reduziert werden.

In der einfachsten Variante beinhaltet die Aquakulturanlage (1) nur einen Kulturbehälter (6) und einen Behälter (22) zur Aufzucht von Futtertieren (3) in einem verkleinerten Wasserkreislauf (13). Die Behälter (6,22) können mehrfach vorhanden sein. Die fluidische Fördertechnik kann vereinfacht sein und ggf. ohne Pumpensumpf auskommen. Der Pflanzenfilter (8) und der Algenreaktor (9) fehlen. Eine evtl. Filterung oder sonstige Aufbereitung des Kulturwassers (5) kann auf andere und ggf. konventionelle Weise, z.B. mechanisch und/oder biologisch mit Mikroorganismen, geschehen. Der Behälter (22) dient vorrangig der kultur- und zuchtnahen Futtertierproduktion. Dabei kann ggf. ein kleiner Filterkreislauf (14) und/oder ein kleiner Fütterkreislauf (15) gebildet werden.

In einer anderen Ausbaustufe der Aquakulturanlage (1) werden der Kulturbehälter (6) und der Behälter (22) mit dem Pflanzenfilter (8) in einem erweiterten Filterkreislauf (14) verbunden, wobei ebenfalls eine Mehrfachanordnung von Behältern (6,22,23) möglich ist. Der Algenreaktor (9) kann entfallen.

Die nächste Variante sieht eine Kombination des Kulturbehälters (6) und des Behälters (22) mit dem Algenreaktor (9), ggf. in mehrfacher Behälteranordnung, vor. Hierdurch wird ein erweiterter Fütterkreislauf (15) gebildet. Der Pflanzenfilter (8) entfällt. Eine evtl. Filterung oder sonstige Aufbereitung des Kulturwassers (5) kann auf andere und ggf. konventionelle Weise, z.B. mechanisch und/oder biologisch mit Mikroorganismen, erfolgen.

Variabel ist auch die Zahl und Anordnung der vorbeschriebenen Komponenten der Aquakulturanlage (1). Auf den vertikalen Aufbau kann zugunsten einer horizontalen Anordnung verzichtet werden. Ferner kann eine andere fluidischen Verbindungs- und Fördertechnik eingesetzt werden.

### BEZUGSZEICHENLISTE

- 1: Aquakulturanlage, Zuchtanlage
- 2: aquatisches Lebewesen, Fisch
- 3: Futtertier, mariner Wurm, Borstenwurm, Seeringelwurm
- 4: Pflanze, Halophyt, Queller
- 5: Kulturwasser, Prozesswasser
- 6: Kulturbehälter, Fischtank
- 7: Filter partikulär, Futtertierfilter, Wurmfilter
- 8: Filter gelöste Ausscheidung, Pflanzenfilter
- 9: Algenreaktor
- 10: Wasserzufuhr
- 11: Wasserabfuhr
- 12: Fluidverbindung, Leitung
- 13: Kreislauf, Wasserkreislauf
- 14: Kreislauf, Filterkreislauf
- 15: Kreislauf, Fütterkreislauf
- 16: Pumpensumpf
- 17: Pumpe, Förderpumpe
- 18: Pumpensumpf
- 19: Pumpe, Förderpumpe
- 20: Wasseraufbereitung, Sauerstoffanreicherung
- 21: Fluidtransport diskontinuierlich
- 22: Behälter für Futtertiere
- 23: Behälter für Pflanzenfilter
- 24: Rückführleitung
- 25: Rückführleitung
- 26: Rückführleitung
- 27: Reaktorbehälter
- 28: Substratschicht, Substratboden, Sandboden
- 29: Fluidablauf
- 30: Ablaufrohr
- 31: Siphon
- 32: Auslauföffnung
- 33: Fluidzulauf
- 34: Fluidüberleitung
- 35: Hydroponik, Hydrokultur
- 36: Flutungseinrichtung
- 37: Belüftungseinrichtung
- 38: Luftzufuhr, Luftdüse
- 39: Luftabfuhr, Ablassventil
- 40: Algensuspension
- 41: Wasserstand, Wasserspiegel
- 42: Behälterdeckel
- 43: Reaktormodul
- 44: Steuerung
- 45: Rohr, Glasrohr
- 46: Leuchteinheit, LED-Platinen
- 47: Kühlkörper
- 48: Überdruckventil
- 49: Regal
- 50: Fördermittel, Gabelstapler

## Patentansprüche

1. Aquakulturanlage für die Aufzucht von aquatischen Lebewesen (2), insbesondere Fischen, mit einem Kulturbehälter (6) für aquatische Lebewesen (2), der über eine Fluidverbindung (12) des Kulturwassers (5) mit mehreren in einem Stapel angeordneten Behältern (22) verbunden ist, die zur Aufzucht von Futtertieren (3), insbesondere von marinen Würmern, bevorzugt Borstenwürmern (Annelida) bzw. Seeringelwürmern (Nereididae), sowie als Futtertierfilter (7), insbesondere als Wurmfilter, für partikuläre Ausscheidungen der aquatischen Lebewesen (2) vorgesehen und ausgebildet sind, wobei die Behälter (22) zur Aufzucht von Futtertieren (3) über eine Fluidverbindung (12) mit einem Algenreaktor (9) verbunden sind, wobei der Behälter (22) zur Aufzucht von Futtertieren (3) und ggf. der Kulturbehälter (6) eine Substratschicht (28), insbesondere eine Sandschicht, mit darin enthaltenen Futtertieren (3), insbesondere marinen Würmern, bevorzugt Borstenwürmern bzw. Seeringelwürmern, aufweist und wobei die Behälter (22) zur Aufzucht von Futtertieren (3) eine Flutungseinrichtung (36) zur Veränderung des Wasserstands, insbesondere zur Simulation von Ebbe und Flut, aufweisen.

2. Aquakulturanlage nach Anspruch 1, **dadurch gekennzeichnet, dass** die Behälter (22) zur Aufzucht von Futtertieren (3) über eine Fluidverbindung (12) des Kulturwassers (5) mit einem Filter (8), insbesondere einem Pflanzenfilter, für gelöste Ausscheidungen der aquatischen Lebewesen (2) verbunden sind.

3. Aquakulturanlage nach Anspruch 2, **dadurch gekennzeichnet, dass** der Pflanzenfilter (8) einen oder mehrere mit Kulturwasser (5) befüllten Behälter (23) mit Pflanzen (4), insbesondere in einer Hydroponik (35) oder Hydrokultur, aufweist.

4. Aquakulturanlage nach Anspruch 3, **dadurch gekennzeichnet, dass** die Behälter (22) zur Aufzucht von Futtertieren (3) und/oder die Behälter (23) mit Pflanzen (4) in einer Turm- oder Kaskadenanordnung angeordnet sind, wobei das Kulturwasser die verschiedenen Behälter (22,23) nacheinander und durch Schwerkraft durchfließt.

5. Aquakulturanlage nach Anspruch 3 oder 4, **dadurch gekennzeichnet, dass** der oder die Behälter (23) mit Pflanzen (4) eine Flutungseinrichtung (36) zur Veränderung des Wasserstands relativ zu den Pflanzen (4), insbesondere zur Simulation von Ebbe und Flut, aufweisen.

6. Aquakulturanlage nach einem der Ansprüche 3 bis 5, **dadurch gekennzeichnet, dass** der Kulturbehälter (6), der Behälter (22) zur Aufzucht von Futtertieren (3), der Behälter (23) mit Pflanzen (4) und der Algenreaktor (9) in einen Wasserkreislauf (13) des Kulturwassers (5) eingebunden sind.

7. Aquakulturanlage nach einem der Ansprüche 3 bis 6, **dadurch gekennzeichnet, dass** der Kulturbehälter (6) und/oder der Behälter (22) zur Aufzucht von Futtertieren (3) und/oder der Behälter (23) mit Pflanzen (4) einen Fluidablauf (29) mit einem in den Behälter (6,22,23) ragenden Ablaufrohr (30) und einem Siphon (31) aufweisen.

8. Aquakulturanlage nach einem der Ansprüche 3 bis 7, **dadurch gekennzeichnet, dass** die Aquakulturanlage (1) mit salzhaltigem Kulturwasser (5) betrieben wird, wobei die Futtertiere (3) als marine Würmer, bevorzugt Borstenwürmer bzw. Seeringelwürmer, und die Pflanzen (4) als Halophyten, bevorzugt Queller, ausgebildet sind.

9. Aquakulturanlage nach einem der vorhergehenden Ansprüche, **dadurch gekennzeichnet, dass** der Algenreaktor (9) als Photobioreaktor für die Mikroalgenzucht in einer Algensuspension (40) ausgebildet, wobei den Algen Nährstoffe und Licht für die Photosynthese zugeführt werden.

10. Verfahren für die Kultivierung von aquatischen Lebewesen (2), insbesondere Fischen, in einer Aquakulturanlage (1) mit einem Kulturbehälter (6) für aquatische Lebewesen (2), wobei dessen Kulturwasser (5) über eine Fluidverbindung (12) zu mehreren in einem Stapel angeordneten Behältern (22) geleitet wird, die zur Aufzucht von Futtertieren (3), insbesondere von marinen Würmern, bevorzugt Borstenwürmern (Annelida) bzw. Seeringelwürmern (Nereididae), vorgesehen und ausgebildet sind und als Futtertierfilter (7), insbesondere als Wurmfilter, partikuläre Ausscheidungen der aquatischen Lebewesen (2) ausfiltern, wobei die Behälter (22) zur Aufzucht von Futtertieren (3) über eine Fluidverbindung (12) mit einem Algenreaktor (9) verbunden werden, wobei
der Behälter (22) zur Aufzucht von Futtertieren (3) und ggf. der Kulturbehälter (6) eine Substratschicht (28), insbesondere eine Sandschicht, mit darin enthaltenen Futtertieren (3), insbesondere marinen Würmern, bevorzugt Borstenwürmern bzw. Seeringelwürmern, aufweist und wobei im Futtertierfilter (7) der Wasserstand verändert wird, wobei Ebbe und Flut simuliert werden.

11. Verfahren nach Anspruch 10, **dadurch gekennzeichnet, dass** die Futtertiere (3) mit Mikroalgen aus dem Algenreaktor (9) gefüttert werden und/oder die aquatischen Lebewesen (2), insbesondere Fische, mit den Futtertieren (3) gefüttert werden.

12. Verfahren nach Anspruch 10 oder 11, **dadurch gekennzeichnet, dass** aus dem Kulturwasser (5) die gelösten Ausscheidungen der aquatischen Lebewesen (2), insbesondere Fische, durch einen Pflanzenfilter (8) ausgefiltert werden.

13. Verfahren nach Anspruch 12, **dadurch gekennzeichnet, dass** im Pflanzenfilter (8) der Wasserstand verändert wird, wobei Ebbe und Flut simuliert werden.

14. Verfahren nach einem der Ansprüche 10 bis 13, **dadurch gekennzeichnet, dass** die Aquakulturanlage (1) mit Salzwasser betrieben wird.

15. Verfahren nach einem der Ansprüche 10 bis 14, **dadurch gekennzeichnet, dass** das Kulturwasser (5) in einem geschlossenen Wasserkreislauf (13) geführt und umgewälzt wird.

16. Verfahren nach einem der Ansprüche 10 bis 14, **dadurch gekennzeichnet, dass** den aquatischen Lebewesen (2) und den Futtertieren (3) bedarfsweise anderes und zusätzliches Futter zugeführt wird.

## Claims

1. Aquaculture system for the breeding of aquatic lifeforms (2), especially fish, having a culture vessel (6) for aquatic lifeforms (2) that is connected via a fluid connection (12) of the culture water (5) to multiple vessels (22) arranged in a stack that are intended and designed for breeding of feed animals (3), especially of marine worms, preferably bristleworms (Annelida) or ragworms (Nereididae), and also as feed animal filter (7), especially as worm filter, for particulate excretions of the aquatic lifeforms (2), wherein the vessels (22) for breeding of feed animals (3) are connected via a fluid connection (12) to an algae reactor (9), wherein the vessel (22) for breeding of feed animals (3) and optionally the culture vessel (6) has a substrate layer (28), especially a sand layer, with feed animals (3) present therein, especially marine worms, preferably bristleworms or ragworms, and wherein the vessels (22) for breeding of feed animals (3) have a flooding device (36) for changing the water level, especially for simulation of ebb and flow.

2. Aquaculture system according to Claim 1, **characterized in that** the vessels (22) for breeding of feed animals (3) are connected via a fluid connection (12) of the culture water (5) to a filter (8), especially a plant filter, for dissolved excretions of the aquatic lifeforms (2).

3. Aquaculture system according to Claim 2, **characterized in that** the plant filter (8) comprises one or more vessels (23) filled with culture water (5) and containing plants (4), especially in a hydroponic system (35) or hydroculture.

4. Aquaculture system according to Claim 3, **characterized in that** the vessels (22) for breeding of feed animals (3) and/or the vessels (23) containing plants (4) are disposed in a tower or cascade arrangement, wherein the culture water flows through the different vessels (22, 23) successively and by means of gravity.

5. Aquaculture system according to Claim 3 or 4, **characterized in that** the vessel(s) (23) containing plants (4) have a flooding device (36) for changing the water level relative to the plants (4), especially for simulation of ebb and flow.

6. Aquaculture system according to any of Claims 3 to 5, **characterized in that** the culture vessel (6), the vessel (22) for breeding feed animals (3), the vessel (23) containing plants (4) and the algae reactor (9) are incorporated into a water circuit (13) of the culture water (5).

7. Aquaculture system according to any of Claims 3 to 6, **characterized in that** the culture vessel (6) and/or the vessel (22) for breeding feed animals (3) and/or the vessel (23) containing plants (4) have a fluid outflow (29) with an outflow tube (30) that projects into the vessel (6, 22, 23) and a siphon (31) .

8. Aquaculture system according to any of Claims 3 to 7, **characterized in that** the aquaculture system (1) is operated with saline culture water (5), wherein the feed animals (3) are marine worms, preferably bristleworms or ragworms, and the plants (4) are halophytes, preferably salsola.

9. Aquaculture system according to any of the preceding claims, **characterized in that** the algae reactor (9) takes the form of a photobioreactor for the breeding of microalgae in an algae suspension (40), wherein the algae are supplied with nutrients and light for photosynthesis.

10. Method for the cultivation of aquatic lifeforms (2), especially fish, in an aquaculture system (1) having a culture vessel (6) for aquatic lifeforms (2), wherein the culture water (5) thereof is connected via a fluid connection (12) to multiple vessels (22) arranged in a stack that are intended and designed for breeding of feed animals (3), especially of marine worms, preferably bristleworms (Annelida) or ragworms (Nereididae), and as feed animal filter (7), especially as worm filter, filter out particulate excretions of the aquatic lifeforms (2), wherein the vessels (22) for breeding of feed animals (3) are connected via a fluid connection (12) to an algae reactor (9), wherein
the vessel (22) for breeding of feed animals (3) and optionally the culture vessel (6) has a substrate layer (28), especially a sand layer, with feed animals (3) present therein, especially marine worms, preferably bristleworms or ragworms, and wherein the water level in the feed animal filter (7) is varied, simulating ebb and flow.

11. Method according to Claim 10, **characterized in that** the feed animals (3) are fed with microalgae from the algae reactor (9) and/or the aquatic lifeforms (2), especially fish, are fed with the feed animals (3) .

12. Method according to Claim 10 or 11, **characterized in that** the dissolved excretions of the aquatic lifeforms (2), especially fish, are filtered out of the culture water (5) by means of a plant filter (8) .

13. Method according to Claim 12, **characterized in that** the water level in the plant filter (8) is varied, simulating ebb and flow.

14. Method according to any of Claims 10 to 13, **characterized in that** the aquaculture system (1) is operated with salt water.

15. Method according to any of Claims 10 to 14, **characterized in that** the culture water (5) is guided and circulated in a closed water circuit (13) .

16. Method according to any of Claims 10 to 14, **characterized in that** the aquatic lifeforms (2) and the feed animals (3) are supplied with other and additional feed as required.

## Revendications

1. Installation d'aquaculture destinée à l'élevage d'organismes aquatiques (2), en particulier de poissons, ladite installation comprenant un récipient de culture (6), destiné à des organismes aquatiques (2), lequel est relié par une liaison fluidique (12) d'eau de culture (5) à une pluralité de récipients (22) qui sont disposés en empilement et qui sont prévus et conçus pour l'élevage d'animaux (3) destinés à l'alimentation animale, en particulier des vers marins, de préférence des annélides (annelida) ou des néréides (nereididae), et comme filtre (7) pour animaux destinés à l'alimentation animale, en particulier comme filtre à vers, pour les excrétions de particules d'organismes aquatiques (2), les récipients (22) destinés à l'élevage d'animaux (3) destinés à l'alimentation animale étant reliés à un réacteur à algues (9) par le biais d'une liaison fluidique (12), le récipient (22) destiné à l'élevage d'animaux (3) destinés à l'alimentation animale et, le cas échéant, le récipient de culture (6) comportant une couche de substrat (28), en particulier une couche de sable, contenant des animaux (3) destinés à l'alimentation animale, en particulier des vers marins, de préférence des annélides ou des néréides et les récipients (22) destinés à l'élevage d'animaux (3) destinés à l'alimentation animale possèdent un dispositif de mise en eau (36) destiné à faire varier le niveau de l'eau, en particulier simuler le flux et le reflux.

2. Installation d'aquaculture selon la revendication 1, **caractérisée en ce que** les récipients (22) destinés à l'élevage d'animaux (3) destinés à l'alimentation animale sont reliés par le biais d'une liaison fluidique (12) d'eau de culture (5) à un filtre (8), notamment un filtre végétal, destinés aux excrétions dissoutes des organismes aquatiques (2).

3. Installation d'aquaculture selon la revendication 2, **caractérisée en ce que** le filtre végétal (8) comporte un ou plusieurs récipients (23) remplis d'eau de culture (5) et comprenant des plantes (4), notamment en hydroponie (35) ou en hydroculture.

4. Installation d'aquaculture selon la revendication 3, **caractérisée en ce que** les récipients (22) destinés à l'élevage d'animaux (3) destinés à l'alimentation animale et/ou les récipients (23) comprenant des plantes (4) sont disposés selon un agencement en tour ou en cascade, l'eau de culture s'écoulant à travers les différents récipients (22, 23) successivement et par gravité.

5. Installation d'aquaculture selon la revendication 3 ou 4, **caractérisée en ce que** le ou les récipients (23) comprenant des plantes (4) comportent un dispositif de mise en eau (36) destiné à modifier le niveau d'eau par rapport aux plantes (4), notamment simuler le flux et le reflux.

6. Installation d'aquaculture selon l'une des revendications 3 à 5, **caractérisée en ce que** le récipient de culture (6), le récipient (22) destiné à l'élevage d'animaux (3) destinés à l'alimentation animale, le récipient (23) comprenant des plantes (4) et le réacteur à algues (9) sont intégrés dans un circuit (13) d'eau de culture (5) .

7. Installation d'aquaculture selon l'une des revendications 3 à 6, **caractérisée en ce que** le récipient de culture (6) et/ou le récipient (22) destinés à l'élevage d'animaux (3) destinés à l'alimentation animale et/ou le récipient (23) comprenant des plantes (4) comportent une évacuation de fluide (29) pourvue d'un tube d'évacuation (30) faisant saillie dans le récipient (6, 22, 23) et d'un siphon (31) .

8. Installation d'aquaculture selon l'une des revendications 3 à 7, **caractérisée en ce que** l'installation d'aquaculture (1) fonctionne avec de l'eau de culture saline (5), les animaux (3) destinés à l'alimentation animale étant des vers marins, de préférence des annélides ou des néréides, et les plantes (4) étant des halophytes, de préférence la salicorne.

9. Installation d'aquaculture selon l'une des revendications précédentes, **caractérisée en ce que** le réacteur à algues (9) est conçu comme un photobioréacteur destiné à la culture de microalgues dans une suspension d'algues (40), des nutriments et de la lumière étant amenés aux algues pour la photosynthèse.

10. Procédé de culture d'organismes aquatiques (2), en particulier de poissons, dans une installation d'aquaculture (1) comprenant un récipient de culture (6) destiné à des organismes aquatiques (2), l'eau de culture (5) étant amenée par le biais d'une liaison fluidique (12) à une pluralité de récipients (22) disposées en empilement, lesquels sont prévus et conçus pour l'élevage d'animaux (3) destinés à l'alimentation animale, en particulier des vers marins, de préférence des annélides (Annelida) ou des néréides (Nereididae) et qui filtrent les excrétions de particules des organismes aquatiques (2) en tant que filtres pour animaux (7) destinés à l'alimentation animale, en particulier en tant que filtres à vers, le récipient (22) destiné à l'élevage d'animaux (3) destinés à l'alimentation animale étant relié par le biais d'une liaison fluidique (12) à un réacteur à algues (9), le récipient (22) destiné à l'élevage d'animaux (3) destinés à l'alimentation animale et, le cas échéant, les récipients de culture (6) comportant une couche de substrat (28), en particulier une couche de sable, contenant des animaux (3) destinés à l'alimentation animale, en particulier des vers marins, de préférence des annélides ou néréides et le niveau d'eau étant modifié dans le filtre (7) destiné aux animaux destinés à l'alimentation animale, le flux et le reflux étant simulés.

11. Procédé selon la revendication 10, **caractérisé en ce que** les animaux (3) destinés à l'alimentation animale sont nourris avec des microalgues du réacteur à algues (9) et/ou les organismes aquatiques (2), notamment des poissons, sont nourris avec des animaux (3) destinés à l'alimentation animale.

12. Procédé selon la revendication 10 ou 11, **caractérisé en ce que** les excrétions dissoutes des organismes aquatiques (2), notamment des poissons, sont filtrées de l'eau de culture (5) par un filtre végétal (8) .

13. Procédé selon la revendication 12, **caractérisé en ce que** le niveau d'eau est modifié dans le filtre végétal (8), le flux et le reflux pouvant être simulés.

14. Procédé selon l'une des revendications 10 à 13, **caractérisé en ce que** l'installation d'aquaculture (1) fonctionne avec de l'eau salée.

15. Procédé selon l'une des revendications 10 à 14, **caractérisé en ce que** l'eau de culture (5) est guidée et mise en circulation dans un circuit d'eau fermé (13) .

16. Procédé selon l'une des revendications 10 à 14, **caractérisé en ce que** si nécessaire d'autres aliments complémentaires sont amenés aux organismes aquatiques (2) et aux animaux (3) destinés à l'alimentation animale.
